# EUROPEAN PATENT APPLICATION

(11) **EP 1 700 611 A1**
(43) Date of publication of application: **13.09.2006**
(21) Application number: 05002937.0
(22) Date of filing: 11.02.2005
(51) Int. Cl.: A61L 27/54, A61L 29/16, A61L 31/16, A61L 27/34, A61L 29/08, A61L 31/10, A61Q 11/00, A61C 13/00

(54) **Device for treatment of disorders in the oral cavity, and manufacturing process for the same**

(71) Applicant: NOLabs AB, 252 21 Helsingborg (SE)
(72) Inventor: Peters, Tor, 8703 Erlenbach (CH)
(74) Representative: Karlsson, Leif Gunnar Börje

(57) **Abstract**

A device and method for therapeutical treatment of disorders in the oral cavity and a process for manufacturing of said device is disclosed. The device being comprised of a nitric oxide (NO) eluting polymer. The nitric oxide (NO) eluting polymer is configured for eluting a therapeutic dosage of nitric oxide (NO) when used in the oral cavity. The device allows for target treatment of infections or wounds in the oral cavity. The device comprising the nitric oxide (NO) eluting polymer is arranged to contact an infected area in the oral cavity, such that a therapeutic dose of nitric oxide is eluted from said nitric oxide eluting polymer to said area.

## Description

### Field of the Invention

This invention pertains in general to the field of therapeutical treatment of disorders in the oral cavity. More particularly the invention relates to a device and method of treatment of disorders in the oral cavity and a process for manufacturing of said device, involving Nitric Oxide (NO).

### Background of the Invention

Infections by bacteria, viruses, fungi or yeasts are the underlying cause of many complications during wound care. A wide range of treatments has been developed to control such disorders, including physical and chemical methods and antimicrobial agents of a wide variety of antimicrobial agents. Despite the widespread use of these approaches, it is generally recognised that our ability to halt the invasion, persistence and spread of microbial infections remain limited.

Treatment of disorders in the oral cavity, such as paradontosis, is especially difficult, since the mucous membrane in the oral cavity is exposed to a variety of substances from the external (outside of the body) environment. Up to now the only reliable treatment of infections in the oral cavity appears to be the use of antibiotics. Treatment with antibiotics has certain disadvantages, such as that the bacteria develops tolerance and resistance to the antibiotics over time, and thus become difficult to eradicate.

It is known that nitric oxide (NO) provides an alternative to conventional therapies, such as antibiotics. Nitric oxide is a highly reactive molecule that is involved in many cell functions. In fact, nitric oxide plays a crucial role in the immune system and is utilized as an effector molecule by macrophages to protect itself against a number of pathogens, such as fungi, viruses, bacteria etc., and general microbial invasion. This improvement of healing is partly caused by NO inhibiting the activation or aggregation of blood platelets, and also by NO causing a reduction of inflammatory processes at the site of an implant. Furthermore, NO has a vasodilating effect, which effect also affect, and promote, the healing process.

NO is also known to have an anti-pathogenic, especially an anti-viral, effect, an anti-sacral effect, and furthermore NO has an anti-cancerous effect, as it is cytotoxic and cytostatic in therapeutic concentrations, i.e. it has among other effects tumoricidal and bacteriocidal effects. NO has for instance cytotoxic effects on human haematological malignant cells from patients with leukaemia or lymphoma, whereby NO may be used as a chemotherapeutic agent for treating such haematological disorders, even when the cells have become resistant to conventional anti-cancer drugs. This anti-pathogenic and anti-tumour effect of NO is taken advantage of by the present invention, without having adverse effects as for instance many anti-cancer drugs.

However, due to the short half-life of NO, it has hitherto been very hard to treat viral, bacteria, virus, fungi or yeast infections with NO. This is because NO is actually toxic and has negative effects when applied in too large amounts to the body. NO is actually also a vasodilator, and too large amounts of NO introduced into the body will cause a complete collapse of the circulatory system. On the other hand, NO has a very short half-life of fractions of a second up to a few seconds, once it is released. Hence, administration limitations due to short half-life and toxicity of NO have been limiting factors in the use of NO in the field of anti-pathogenic and anti-cancerous treatment so far.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

Other example for NO eluting polymers are given in US-5,770,645, wherein polymers derivatized with at least one -NOₓ group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups.

Akron University has developed NO-eluting L-PEI molecule that can be nano-spun onto the surface of medical devices such as implanted grafts, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner to tissues and organs to aid the healing process and to prevent injury to tissues at risk of injury. Electrospun nano-fibers of linear poly(ethylenimine) diazeniumdiolate deliver therapeutic levels of NO to the tissues surrounding a medical device while minimizing the alteration of the properties of the device. A nanofiber coating, because of the small size and large surface area per unit mass of the nanofibers, provides a much larger surface area per unit mass while minimizing changes in other properties of the device.

However, the disclosure is both silent concerning an improvement of present technology in respect of treatment of disorders in the oral cavity, and the anti pathogenic potential of nitric oxide.

Hence, an improved device for the treatment and/or prevention of infection, caused by bacteria, viruses, fungi or yeasts, herpes, which device does not develop bacteria resistance, do increase circulation, and acts as a healing promoter, would be advantageous, and in particular a device allowing for target treatment of both osteosynthetic and soft tissue healing post dental implant, prevention and treatment of paradontosis or other infected wounds and cancer, and increased circulation, in the oral cavity would be advantageous.

### Summary of the Invention

Accordingly, the present invention preferably seeks to mitigate, alleviate or eliminate one or more of the above-identified deficiencies in the art and disadvantages singly or in any combination and solves at least the problems mentioned above, by providing a device according to the appended patent claims.

According to one aspect of the invention, a device is provided that allows for target treatment of infections or wounds in the oral cavity. The device comprises a nitric oxide (NO) eluting polymer arranged to contact the infected area in the oral cavity, such that a therapeutic dose of nitric oxide is eluted from said nitric oxide eluting polymer to said area.

According to another aspect of the invention, a manufacturing process for such a device is provided, wherein the process is a process for forming a device that allows for target treatment of infections or wounds in the oral cavity. The process comprises selecting a plurality of nitric oxide eluting polymeric fibers, and deploying said nitric oxide eluting fibers in a patch or pad to be comprised in said device.

The present invention has at least the advantage over the prior art that it provides target exposure of an infected or wounded area to NO, whereby a very effective anti-viral, anti-bacterial, anti-fungi and/or anti-cancer therapy is achievable.

### Brief Description of the Drawings

These and other aspects, features and advantages of which the invention is capable of will be apparent and elucidated from the following description of embodiments of the present invention, reference being made to the accompanying drawings, in which
Fig. 1 is a schematic illustration of a sponge according to the invention,
Fig. 2 is a schematic illustration of a patch or pad according to the invention,
Fig. 3 is a schematic illustration of another patch or pad according to the invention, with NO-elution in one direction only,
Fig. 4 is a schematic illustration of a condom/sheath according to the invention,
Fig. 5 is a schematic illustration of nano-particles, or micro-spheres, according to the invention, and
Fig. 6 is a schematic illustration of a mouth wash according to the invention.

### Description of Embodiments

The following description focuses on an embodiment of the present invention applicable to a device, in form of a pad or patch, which allows for target treatment of infections or wounds in the oral cavity, such as paradontosis, herpes etc. However, also alternative embodiments are described.

With regard to nitric oxide (nitrogen monoxide, NO), its physiological and pharmacological roles have attracted much attention and thus have been studied. NO is synthesized from arginine as the substrate by nitric oxide synthase (NOS). NOS is classified into a constitutive enzyme, cNOS, which is present even in the normal state of a living body and an inducible enzyme, iNOS, which is produced in a large amount in response to a certain stimulus. It is known that, as compared with the concentration of NO produced by cNOS, the concentration of NO produced by iNOS is 2 to 3 orders higher, and that iNOS produces an extremely large amount of NO.

In the case of the generation of a large amount of NO as in the case of the production by iNOS, it is known that NO reacts with active oxygen to attack exogenous microorganisms and cancer cells, but also to cause inflammation and tissue injury. On the other hand, in the case of the generation of a small amount of NO as in the case of the production by cNOS, it is considered that NO takes charge of various protective actions for a living body through cyclic GMP (cGMP), such as vasodilator action, improvement of the blood circulation, antiplatelet-aggregating action, antibacterial action, anticancer action, acceleration of the absorption at the digestive tract, renal function regulation, neurotransmitting action, erection (reproduction), learning, appetite, and the like. Heretofore, inhibitors of the enzymatic activity of NOS have been examined for the purpose of preventing inflammation and tissue injury, which are considered to be attributable to NO generated in a large amount in a living body. However, the promotion of the enzymatic activity (or expressed amount) of NOS (in particular, cNOS) has not been examined for the purpose of exhibiting various protective actions for a living body by promoting the enzymatic activity of NOS and producing NO appropriately.

In recent years research has been directed to polymers with the capability of releasing nitrogen oxide when getting in contact with water. Such polymers are for example polyalkyleneimines, such as L-PEI (Linear PolyEthyleneImine) and B-PEI (Branched PolyEthyleneImine), which polymers have the advantage of being biocompatible, after the release of nitrogen oxide.

The polymers according to embodiments of the present invention may be manufactured by electro spinning. Electro spinning is a process by which a suspended polymer is charged. At a characteristic voltage a fine jet of polymer releases from the surface in response to the tensile forces generated by interaction by an applied electric field with the electrical charge carried by the jet. This process produces a bundle of polymer fibres, such as nano-fibres. This jet of polymer fibres may be directed to a surface to be treated.

Furthermore, US 6,382,526, US 6,520,425, and US 6,695,992 disclose alternative processes and apparatuses for the production of such polymeric fibres. These techniques are generally based on gas stream spinning, also known within the fiber forming industry as air spinning, of liquids and/or solutions capable of forming fibers.

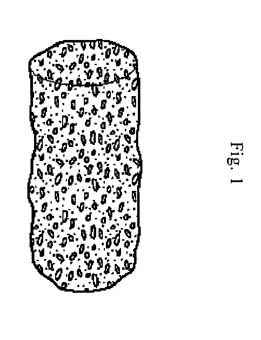

In an embodiment of the invention, according to Fig. 1, the device according to the present invention is in form of a nano-spun, NO-eluting sponge or fiber coated sponge or sponge-like device, such as a sponge or a cotton ball or pillow. This NO-eluting sponge may be placed between the lip and teeth to increase circulation and prevent infection. When the nano-spun, NO-eluting sponge according to the present invention gets in contact with the moisture in the oral cavity the NO-eluting sponge starts to release NO to the area to be treated. This nano-spun NO-eluting sponge is preferably comprised of nano-spun fibres of a polymer that elutes NO. Such polymers are for example polyalkyleneimines, such as L-PEI (Linear PolyEthyleneImine) or B-PEI (Branched PolyEthyleneImine), which polymers have the advantage of being biodegradable to natural products or biocompatible with the latter, after the release of nitrogen oxide.

This sponge has the advantage that it is easily activated. Furthermore, the NO is applied locally, without influencing other parts of the body, due to the short half-life of the NO eluted from the NO eluting polymer material. Thus, implications concerning the vascular system are kept very local and low, while at the same time the effect of NO is optimally exploited.

In another embodiment the sponge according to an embodiment of the present invention is applied to the oral cavity with the aid of a stick or pin at the area to be treated. This area may be anywhere in the oral cavity, such as between the gum and the teeth, between the teeth, in a dental pocket, etc. More sepcifically, the sponge is for instance releasably attached to the stick, preferably to an end of the stick. The end is then introduced into the oral cavity together with the sponge, where it is released from the stick, e.g. by counter holding the sponge with two fingers and drawing back the stick. The stick is thus removed from the oral cavity, leaving the sponge behind in the oral cavity for treatment therein.

Akron University has developed NO-eluting L-PEI molecule that can be nano-spun onto the surface of medical devices permanently implantable into the human body, showing significant improvement of the healing process and reduced inflammation when implanting such devices. According to US-6,737,447, a coating for medical devices provides nitric oxide delivery using nanofibers of linear poly(ethylenimine)-diazeniumdiolate. Linear poly(ethylenimine)diazeniumdiolate releases nitric oxide (NO) in a controlled manner.

It is preferable that the nano-spun fibres in the NO-eluting sponge according to the embodiment of present invention comprise L-PEI. This embodiment of course permits the sponge to be placed in another location in the oral cavity than between the lip and the teeth. When placed on an area to be treated the device according to the present invention provides for promotion of osteosynthetic and soft tissue healing, as well as prevention and treatment of paradontosis, infections or wounds, thanks to the effect of NO eluting from the sponge into the regions to be treated. One of the advantages of electrospun or gas-jet spun nanofibres is their large surface area per volume unit. For the sponge this leads to a very effective treatment with a compact device.

One field of application of the device is post dental implant, e.g. accelerated healing thereof, or post-operative infection control, which is simplified and made more effective and convenient by the invention.

Other example for NO eluting polymers are given in US-5,770,645, wherein polymers derivatized with at least one -NOX group per 1200 atomic mass unit of the polymer are disclosed, X being one or two. One example is an S-nitrosylated polymer and is prepared by reacting a polythiolated polymer with a nitrosylating agent under conditions suitable for nitrosylating free thiol groups. Such polymers may also be used for other embodiments of the devices according to the present invention. However, L-PEI is preferred, as the NO is eluted without any secondary products that could lead to undesired side effects as a result of treatment with the devices described herein.

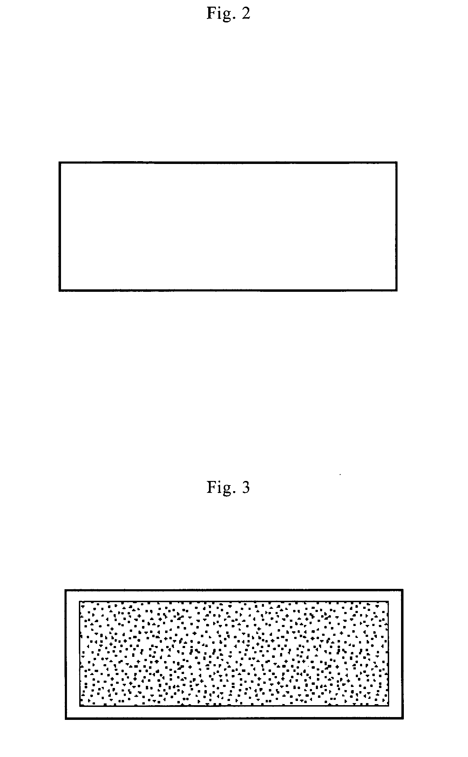

In another embodiment the device according to present invention is in the form of a pad or patch, according to Fig. 2. The pad or patch is coated with or comprises at least partly, at least on one side, nano-spun fibres, which according to embdiments of the present invention comprise the materials mentioned above, regarding the sponge. The nano-spun fibres elute NO in a therapeutic dose as the nano-spun fibres that release NO, which is eluted from the fibres without harmful secondary or waste products, are activated for this purpose when they get in contact with the moisture in the oral cavity. This embodiment has the advantage that it is easily applicable, and removable, on, and from, the target area. Furthermore, this pad or patch has the advantage that it is easily activated. Furthermore, the NO is applied locally, without influencing other parts of the body, due to the short half-life of the NO eluted from the NO eluting polymer material. Alternatively, if the oral cavity of some reason should be deprived of humidity, the patch, and also other devices according to embodiments of the invention, may be activated immediately prior to introduction or in the cavity, e.g. by moisturizing them in a bath of water or by a water sprayed onto the device.

The device according to this embodiment of the present invention may in a further embodiment be soluble in the oral cavity. When the device is subjected to the moisture in the oral cavity, the device is disintegrated in its entirety, wherein the time for dissolving the device is adapted to specific requirements, as for instance therapeutic concentrations to be released over time. This embodiment has the advantage, that it is easily applicable and does not have to be removed for replacement by another device or after the therapeutic treatment with the device is completed.

In another embodiment of the present invention the device only allows NO-elution in one direction, according to Fig. 3. In this kind of embodiment one side of the patch or pad is non-permeable to NO. This may be accomplished by applying a material on one side of the patch or pad that is not permeable to NO. Such materials may be chosen from the group comprising common plastics, such as polyethylene, polyurethane etc. In this way, the therapeutic effect of NO is easily directable to certain regions in the oral cavity without interfering with other regions therein.

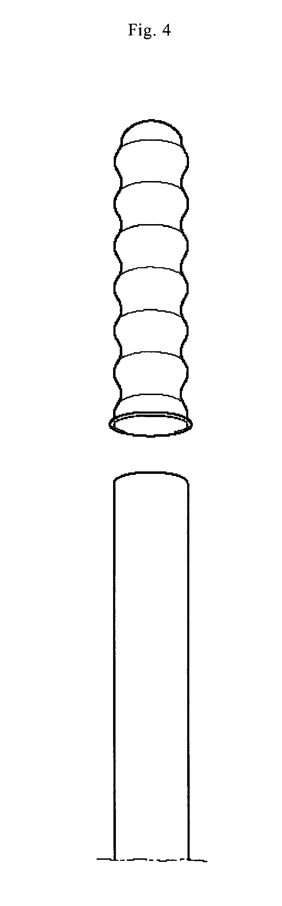

A further embodiment of the invention is illustrated in Fig. 4, in which the device according to the present invention is shaped as a condom/sheath, which either is made of an NO eluting polymer or coated with it, e.g. by nano electro-spinning or gas-jet spinning. According to this embodiment the condom/sheath may be mounted on a stick for easier application. The condom/sheath may be applied by inserting the stick, with the condom/sheath according to the present invention mounted thereon, adjacent to the area to be treated. Then the stick may be extracted from the oral cavity. The condom/sheath is left adjacent to the area to be treated. This area may be areas such as in between the teeth, the tooth pocket, or any other area in the oral cavity where the condom/sheath is applicable.

This condom/sheath may of course be in any suitable size, such as a size suitable for inserting said condom/sheath between the teeth or in a tooth pocket. The condom/sheath is then inserted on the preferred area to be treated with the aid of a suitable means, such as a stick or pin. This embodiment has the advantages that it is easy to pin point the treatment area and it is easy to apply.

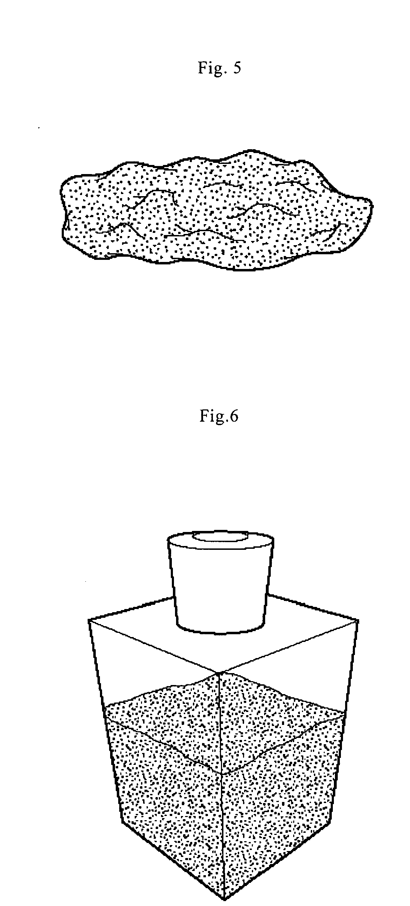

In still another embodiment of the present invention NO-eluting nano-particles, or micro-spheres, may be formed from NO-eluting polymers, according to Fig. 5. These nano-particles, e.g. in the form of micro-spheres may be integrated in a soluble film that disintegrates e.g. in between the lip and the dental soft tissue, in the dental pocket, or any other area in the oral cavity where the device according to the present invention is applicable, in order to elute NO at the area of interest when soluble film gets in contact with the moisture in the oral cavity, or between the inside of the cheek and the gum, in one direction or both.

In another embodiment of the present invention the nano-particles, or micro-spheres, of the polymers in the present invention, may be encapsulated in a material that breaks upon the stress from chewing or brushing the teeth. Then said nano-particles, or micro-spheres, may be integrated in chewing gum or toothpaste. This kind of chewing gum or toothpaste may then be used to prevent or treat disorders in the oral cavity, such as infections, cancer, or paradontosis, or to promote osteosynthesis and soft tissue healing post dental implant. The materials used to encapsulate these nano-particles, or micro-spheres, may be chosen from the group comprising polyvinylacetates, polylacticacids, starch, cellulose, polyhydroxyalkanoates, polyesters, polycaprolactone, polyvinylalcohol, protein based plastics, gelatine, biogradable polymers, and other soluble plastics. This embodiment has the advantages that it is easy to apply, the treatment effect covers the whole oral cavity, and it is easy to manufacture.

In still another embodiment of the present invention the nano-particles, or micro-spheres, may combined with a suitable mouthwash, such as chlorinedioxide (ClO₂), according to Fig. 6. When the mouthwash is used in the oral cavity, the nano-particles, or micro-spheres, break and NO is released. Of course, chlorinedioxide may be combined with all the embodiments according to the present invention, such as the patch. This offers the advantage of further promoting the anti-bacterial effect of NO.

In yet another embodiment of the present invention the NO-eluting device is acting as a booster for drug eluting patches, e.g. pharmaceuticals, vitamins, nicotin, nitroglycerin etc. This embodiment presents a device with the advantage of combining two therapeutic treatments, of significant value, in one treatment. A synergetic effect may be that NO that is eluted from the device has a vasodilatory effect on the region where the device having the combination compound actuates. Vasodilated tissue is more is more susceptible to certain medications and thus more easily treated by the medical preparations and still NO has in addition to that the anti-inflammatory, anti-bacterial etc. effect. Hence, an unexpected surprisingly effective treatment is provided.

In another embodiment of the device according to the present invention the fibres, nano-particles, or micro-spheres may be integrated in a gel, that may either be in a smearing or compressed structure. The fibres, nano-particles, or micro-spheres may also be integrated in a hydrogel, which is mixed directly before use. This embodiment has the advantage of being able to penetrate pockets and corners, e.g in the gum or skin for closer elution of NO on the area to be treated.

In still another embodiment of the present invention dental implants, such as screws of titanium, and other biodegradable or biocompatible plates, may be integrated with the fibres, nano-particles, or micro-spheres according to the present invention, e.g. by coating the devices. A very convenient way for coating is offered by electro-spinning or gas-jet spinning of NO eluting polymers onto the surface of the devices. This embodiment decreases the risk of infections during surgical procedures in the oral cavity.

The device according to the present invention may include materials such as polyesters, polyamides, polyethers, polyurethanes, polycarbonates, polyvinylacetates, polylacticacids, starch, cellulose, polyhydroxyalkanoates, polypropylene, polyacrylonitrile, polystyrene, poly(acrylic acid), cotton, polyesters, polycaprolactone, polyvinylalcohol, protein based plastics, gelatine, and other biocompatible polymers. The NO-eluting polymer may be integrated in, spun together with, or spun on top of, any of these materials in all of the embodiments of the present invention.

The device according to the present invention elutes nitric oxide (NO) from said eluting polymer in a therapeutic dose in the oral cavity, such as between 1 to 100 ppm, such as 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.

In the embodiments of the present invention it may be suitable to control or regulate the time span of NO release from the device according to the invention. This may be accomplished by integrating other polymers or materials in said device. These polymers or materials may be chosen from any suitable material or polymer, such as polyvinylacetates, polylacticacids, starch, cellulose, polyhydroxyalkanoates, polyesters, polycaprolactone, polyvinylalcohol, protein based plastics, gelatine, biogradable polymers, and other soluble plastics.

The sizes of the devices according to the present invention may of course vary widely within in the parameters conveniently used in the oral cavity, but the size is typically 7 to 15 mm X 20 to 40 mm, preferably 9 to 13 mm X 25 to 35 mm, such as 10 mm X 30 mm.

The NO-eluting polymers in the devices according to the present invention may be combined with silver, such as hydroactivated silver. The integration of silver in the devices according to the present invention gives the healing process an extra boost. Preferably the silver is releasable from the devices in the form of silver ions.

The device according to the present invention may be manufactured by, for example electro spinning of L-PEI. L-PEI is the charged at a characteristic voltage, and a fine jet of L-PEI releases as a bundle of L-PEI polymer fibres. This jet of polymer fibres may be directed to a surface to be treated. The surface to be treated may for example be any suitable material in respect of a device according to the present invention. The electro spun fibres of L-PEI then attach on said material and form a coating/layer of L-PEI on the device according to the invention.

It is of course possible to electro spin the other NO-eluting polymers, according to above, on the device according to the invention while still being inside the scope of the present invention.

In one embodiment the NO-eluting polymers according to the present invention are elctro spun in such way that pure NO-eluting polymer fibres may be obtained.

Gas stream spinning, or air spinning, of said NO-eluting polymers onto the device according to the present invention is also within the scope of the present invention.

The manufacturing process according to the present invention presents the advantages of large contact surface of the NO-eluting polymer fibres with the area to be treated, effective use of NO-eluting polymer, and a cost effective way of producing the device according to the present invention.

The device according to the invention may of course be used in any post surgery treatment to prevent, treat, and/or alleviate any kind of infection or inflammation. Especially to prevent disorders post surgery in the oral cavity. The effects of NO are provided in a convenient way by the device of the invention. Such effects are for instance, as mentioned above, anti-inflammatory, anti-pathogenic, especially anti-viral and anti-bacterial. Furthermroe the anti-cancerous effect of NO may be taken advantage of, e.g. for bone cancer treatment of the jaw.

Hereinafter some potential uses of the present invention are described:
A method of therapeutically treating disorders in the oral cavity, comprising introducing a stick or pin having releasably attached thereto a device according any of claims 1-6 into the oral cavity of a patient, releasing the device, in the oral cavity of the patient, from the stick or pin, thereby contacting an area of treatment in the oral cavity, such that a therapeutic dose of nitric oxide is eluted from said nitric oxide eluting polymer to said area.
The method according to the above, wherein said area of treatment is an infected area in the oral cavity or an area where infection is to be prevented for treatment by the effect of the device.
The method according to the above, wherein said treatment area is a post-operative dental surgery area.
The method according to the above, wherein said treatment area is a tumor area in the oral cavity.
Use of nitric oxide (NO) in a therapeutic dose for the treatment of disorders in the oral cavity.
The invention can be implemented in any suitable form. The elements and components of the embodiments according to the invention may be physically, functionally, and logically implemented in any suitable way. Indeed, the functionality may be implemented in a single unit, in a plurality of units, or as part of other functional units.
Although the present invention has been described above with reference to specific embodiments, it is not intended to be limited to the specific form set forth herein. Rather, the invention is limited only by the accompanying claims and, other embodiments than the specific above are equally possible within the scope of these appended claims.
In the claims, the term "comprises/comprising" does not exclude the presence of other elements or steps. Furthermore, although individually listed, a plurality of means, elements or method steps may be implemented. Additionally, although individual features may be included in different claims, these may possibly advantageously be combined, and the inclusion in different claims does not imply that a combination of features is not feasible and/or advantageous. In addition, singular references do not exclude a plurality. The terms "a", "an", "first", "second" etc do not preclude a plurality. Reference signs in the claims are provided merely as a clarifying example and shall not be construed as limiting the scope of the claims in any way.

## Claims

1. A device configured for therapeutic treatment of disorders in the oral cavity, wherein
said device comprises a nitric oxide (NO) eluting polymer, for eluting a therapeutic dosage of nitric oxide (NO) to an area of treatment in the oral cavity, when used in the oral cavity.

2. Device according to claim 1, wherein said polymer is selected from the group comprising polyalkyleneimines, S-nitrosylated polymer, and
poly(alkylenimine) diazeniumdiolates, or any combination of these.

3. Device according to claim 1, wherein said polymer is L-PEI (linear polyethyleneimine), loaded with nitric oxide (NO), arranged for release of the nitric oxide (NO) in the oral cavity for treatment of disorders therein.

4. Device according to claim 1, in form of a sponge, adapted to be applied in the oral cavity.

5. Device according to claim 1, in form of a pad or patch, adapted to be applied in the oral cavity.

6. Device according to claim 1, in form of a condom/sheath, adapted to be applied in the oral cavity.

7. Device according to any of claims 1 to 6, wherein said device is configured to be applied to the oral cavity by the aid of a stick or pin.

8. Device according to claim 1, in form of a screw of titanium, or other biodegradable or biocompatible plate, adapted to be applied in the oral cavity.

9. Device according to claim 1, wherein said device is configured to disintegrate or at least release nitric oxide (NO) from it in the oral cavity when subjected to moisture or water.

10. Device according to claim 1, wherein said NO-eluting polymer is combined with silver, configured for therapeutical treatment of said area of treatment in the oral cavity.

11. Device according to claim 1, wherein said device comprises a material regulating or controlling NO-elution, such as polyvinylacetates, polylacticacids, starch, cellulose, polyhydroxyalkanoates, polyesters, polycaprolactone, polyvinylalcohol, protein based plastics, gelatine, and/or biogradable polymers.

12. Device according to claims 1 to 11, comprising said polymer in the form of nano-particles or micro-spheres.

13. Device according to claim 12, wherein said nano-particles or micro-spheres are integrated in a toothpaste.

14. Device according to claim 12, wherein said nano-particles or micro-spheres are integrated in a chewing gum.

15. Device according to claim 12, wherein said nano-particles or micro-spheres are integrated in a mouth wash.

16. A manufacturing process for a device configured for therapeutic treatment of disorders in the oral cavity according to claim 1, comprising:
selecting a plurality of nitric oxide eluting polymeric particles, preferably nano fibres, nano particles or micro spheres, and
deploying said nitric oxide eluting particles into a suitable form or as a coating onto a carrier, to form said device,
wherein said deploying comprises electro, air, or gas stream spinning of said fibres.

17. Use of a nitric oxide (NO) eluting polymer for the manufacture of a device for the treatment of disorders in the oral cavity,
wherein nitric oxide is loaded to said device so that the device elutes nitric oxide (NO) from said eluting polymer in a therapeutic dose when used in the oral cavity.

18. Use according to claim 15, wherein said therapeutic dose is 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 51, 52, 53, 54, 55, 56, 57, 58, 59, 60, 61, 62, 63, 64, 65, 66, 67, 68, 69, 70, 71, 72, 73, 74, 75, 76, 77, 78, 79, 80, 81, 82, 83, 84, 85, 86, 87, 88, 89, 90 91, 92, 93, 94, 95, 96, 97, 98, 99, or 100 ppm.
